# FASCICULE DE BREVET EUROPEEN

(11) **EP 0 980 240 B1**
(45) Date de publication et mention de la délivrance du brevet: **19.03.2008**
(21) Numéro de dépôt: 98921551.2
(22) Date de dépôt: 17.04.1998
(51) Int. Cl.: A61K 9/16, A61K 38/09

(54) **COMPOSITIONS PRESENTANT UNE LIBERATION PROLONGEE ET LEUR PROCEDE DE PREPARATION**
ZUSAMMENSETZUNGEN MIT VERZOEGERTER FREISETZUNG UND VERFAHREN ZU DEREN HERSTELLUNG
SUSTAINED-RELEASE COMPOSITIONS AND METHOD FOR PREPARING SAME

(30) Priorité: 18.04.1997 FR 9704837; 25.03.1998 FR 9803666
(43) Date de publication de la demande: 23.02.2000
(73) Titulaire: IPSEN PHARMA BIOTECH, 83870 Signes (FR)
(72) Inventeur: PELLET, Marc, F-27160 Condé sur Iton (FR); ROUME, Chantal, F-83870 Signes (FR)
(74) Mandataire: Bourgouin, André
(86) Numéro de dépôt international: PCT/FR1998/000773
(87) Numéro de publication internationale: WO 1998/047489

(56) Documents cités:
- EP-A- 0 384 752
- EP-A- 0 579 347
- EP-A- 0 709 085
- WO-A-97/26869
- CH-A- 661 206
- DE-A- 4 041 563
- FR-A- 2 693 905
- GB-A- 2 246 514

## Description

L'invention concerne tout d'abord une composition sous forme de microcapsules ou d'implants comprenant un excipient ou un mélange d'excipients polymères ou copolymères biodégradables de viscosité inhérente comprise entre 0,5 dl/g et 1,6 dl/g dans CHCl₃ et au moins une substance active. L'invention concerne également une composition sous forme de microcapsules ou d'implants comprenant au moins un polymère ou un copolymère biodégradable de masse moléculaire élevée et au moins une substance active hydrosoluble présentant une surface spécifique élevée. De telles compositions seront utilisées pour obtenir une libération régulière de la substance active sur une période prolongée pouvant aller jusqu'à plus de trois mois.

Ces compositions et notamment les microcapsules trouvent leur utilisation principale en pharmacie, mais peuvent également être employées dans d'autres domaines, en particulier en agrochimie, c'est à dire dans le domaine phytosanitaire.

L'intérêt de l'administration de principes actifs sous forme de compositions à relargage progressif est connu depuis longtemps, qu'il s'agisse de produits pharmaceutiques classiques, par exemple de stéroïdes, de peptides ou de protéines (voir par exemple le brevet US 3,773,919 de Boswell), ou de produits à usage phytosanitaire. Les formulations adoptées peuvent prendre la forme de microparticules dans lesquelles le principe actif est incorporé dans un polymère ou un copolymère biodégradable tel le copolymère polylactide-co-glycolide (PLGA).

Le document DE 4 041 563 divulge des microcapsules comprenant du PLGA.

Il est apparu que notamment lorsqu'un mode de relargage relativement constant, en tout cas sans interruption, est recherché, mode qualifié par exemple de "monophasique" dans le brevet européen EP 58 481, des polymères de type PLGA de relativement bas poids moléculaire, donc de faible viscosité, sont nécessaires. On peut citer à ce sujet les brevets européens EP 21 234 (voir l'exemple 8.B.2. décrivant un copolymère de viscosité intrinsèque de 0,5 dl/g), EP 52 510 où un copolymère ayant une viscosité de 0,38 dl/g dans l'hexaisofluoropropanol (HFIP) est testé *in vivo*, EP 26 599 qui décrit en exemple des polymères ayant des viscosités de 0,12 à 0,20 dl/g et revendique des polymères ayant une viscosité de 0,08 à 0,30 dl/g. Les polymères décrits dans ces brevets sont présentés comme conduisant à des compositions à relargage constant. Les compositions du brevet EP 26 599 peuvent par exemple contenir des agents de contrôle de fertilité.

Il est d'ailleurs important de noter à cet égard que lors de la procédure d'opposition relative au brevet européen EP 58 481, procédure encore en cours au jour du dépôt de la présente demande, le déposant a limité sa revendication principale à des polymères de faible viscosité (inférieure à 0,3 ou 0,5 dl/g), seuls capables selon le déposant de permettre un relargage de type monophasique.

Par ailleurs, lorsqu'une période de relargage plus longue est recherchée, par exemple supérieure à un mois, des problèmes plus complexes apparaissent et une solution proposée par exemple par le brevet EP 0 302 582 consiste à mélanger plusieurs types de microcapsules constituées par des polymères de viscosités différentes.

Or la demanderesse vient de constater que certains polymères de viscosité élevée peuvent convenir à la préparation de compositions à relargage progressif de longue durée. Il a été aussi constaté que l'utilisation de certains polymères conduit à des compositions ayant un profil de relargage monophasique pendant une durée très longue et sans période initiale ne comportant pas de libération ("dead period"). Il en est particulièrement ainsi de polymères ayant une viscosité inhérente de préférence au moins égale à 0,5 dl/g dans CHCl₃, et de façon plus préférentielle au moins égale à 0,6 ou 0,7 dl/g. Toutefois, la viscosité inhérente de ces polymères n'excédera en principe pas 1,6 dl/g dans CHCl₃, et pourra être inférieure à 1,4 ou 1,2 dl/g. Lesdits polymères seront de préférence des PLGA avec un ratio lactide / glycolide allant de 40 / 60 à 90 / 10, et de préférence d'environ 75 / 25.

Les polymères selon l'invention peuvent être préparés par les méthodes usuelles, notamment par ouverture des cycles lactide ou glycolide. Un tel procédé est décrit par exemple dans le brevet américain US 3,773,919.

Dans la présente invention, on peut également utiliser un mélange de polymères de viscosités élevées différentes, mais on préfère les compositions ne comportant qu'un seul polymère ou copolymère.

L'invention concerne donc tout d'abord une composition sous forme de microcapsules ou d'implants comprenant un excipient ou un mélange d'excipients polymères ou copolymères biodégradables de viscosité inhérente comprise entre 0,5 dl/g et 1,6 dl/g dans CHCl₃ et une substance active ou un mélange de substances actives, ces microcapsules ou implants pouvant libérer la substance active ou le mélange de substances actives sur une période prolongée d'au moins 1 mois, de préférence, d'au moins 2 mois et, de façon plus préférentielle, d'au moins 3 mois.

Par microcapsule, on entend également inclure les microsphères, microparticules, nanocapsules, nanosphères ou nanoparticules. Par polymère, on comprendra un polymère, un copolymère, ou un mélange quelconque de ces entités. Enfin, par substance active, on entend une substance active, un de ses sels ou un de ses précurseurs, ou un mélange quelconque de ces composés.

Des sels de substances actives utilisables pour des compositions selon l'invention comprennent notamment les sels obtenus à partir d'acides organiques comme les acides acétique, malique, tartrique, oxalique, fumarique, citrique, lactique, stéarique, pamoïque, méthanesulfonique ou p-toluènesulfonique, ou à partir d'acides inorganiques comme les acides chlorhydrique, sulfurique, phosphorique ou bromhydrique. De préférence, on utilisera un produit hydrosoluble, obtenu par salification sous forme de cation, avec par exemple l'acide acétique. On peut cependant utiliser un sel insoluble, par exemple un pamoate.

En particulier, l'invention concerne une composition sous forme de microcapsules ou d'implants comprenant un excipient ou un mélange d'excipients polymères ou copolymères biodégradables et une substance active ou un mélange de substances actives, lesdites microcapsules ou lesdits implants pouvant libérer la substance active ou le mélange de substances actives sur une période prolongée pouvant aller jusqu'à trois mois ou plus selon un profil essentiellement monophasique, ladite composition étant caractérisée en ce que :
- lorsque la composition est sous forme de microcapsules les excipients comprennent des copolymères dont la viscosité est comprise entre 0,9 dl/g et 1,6 dl/g dans CHCl₃ et le procédé de préparation desdites microcapsules ne comporte pas d'étape de fusion desdites microcapsules,
- ou bien, lorsque la composition est sous forme d'implants, la viscosité desdits polymères ou copolymères est comprise entre 0,5 dl/g et 1,6 dl/g dans CHCl₃.

De préférence, la viscosité des polymères ou copolymères pour les compositions de l'invention sera au moins égale à 0,9 dl/g dans CHCl₃.

Les polymères ou copolymères utilisables pour l'invention peuvent être notamment des polymères tels ceux de l'acide lactique, l'acide glycolique, l'acide citrique ou l'acide malique, ou bien d'autres polymères biocompatibles comme l'acide poly-β-hydroxybutyrique, les polyorthoesters, les polyorthocarbonates, les polyesters d'acide α-cyanoacrylique, les polyoxalates d'alkylène tels le polyoxalate de triméthylène ou de tétraméthylène, les polyaminoacides, etc. Ils peuvent être aussi des copolymères comme le PLGA, le polystyrène, l'acide polyméthacrylique, des copolymères d'acide méthacrylique et d'acide acrylique, des polyaminoacides, des polymères d'anhydride maléique, l'éthylcéllulose, le nitrocellulose, l'acétylcellulose, etc. Tous ces polymères ou copolymères peuvent être utilisés seuls ou en un mélange quelconque. Les PLGA comprendront en général de 40 à 90 % de lactide et de 10 à 60 % de glycolide. De préférence, on utilisera le D,L-PLGA et, plus préférentiellement, un D,L-PLGA réalisé à partir de 70 à 80 % de DL-lactide et de 20 à 30 % de glycolide. Un PLGA synthétisé à partir de 75 % de DL-lactide et 25 % de glycolide conviendra particulièrement bien pour l'invention.

Un autre polymère particulièrement préféré pour l'invention est le L-PLGA, obtenu à partir de L-lactide et de glycolide. Par rapport au D,L-PLGA de même viscosité, le L-PLGA assure une libération plus lente et représente une alternative aux D,L-PLGA de plus haute viscosité.

D'une façon générale, on préférera les polymères ou copolymères de caractère hydrophile. Ainsi, on préférera en général, aux PLGA obtenus par ouverture de cycle avec des initiateurs hydrophobes, tels ceux de type alcool laurique, ceux obtenus par ouverture de cycle, avec des initiateurs hydrophiles, tels ceux de type acide lactique ou acide glycolique.

Par polymère ou copolymère de caractère hydrophile, on entend un polymère ou un copolymère dont la chaîne terminale est polaire (par exemple, cette chaîne terminale comporte à son extrémité une fonction acide), par opposition à un polymère ou copolymère de caractère hydrophobe pour lequel la chaîne terminale est apolaire (par exemple, cette chaîne terminale est une chaîne aliphatique).

L'indice d'acide, correspondant au nombre de milliéquivalents de KOH nécessaires par gramme de polymère pour neutraliser l'acidité libre, semble être le paramètre le mieux corrélé au caractère hydrophile ou hydrophobe d'un polymère ou copolymère. L'indice d'acide pourra être mesuré dès que du fait de la nature du monomère, les chaînes terminales des polymères ou copolymères pourront comporter une fonction acide libre.

D'une façon générale, la demanderesse a trouvé que les polymères hydrophiles donnent un meilleur profil de libération. Ainsi, l'indice d'acide pour les polymères de l'invention sera de préférence au moins égal à 1, ou, mieux, 1,2, et plus préférentiellement au moins égal à 1,5 ou 2.

La charge en substance active ("core loading") des microcapsules selon l'invention, c'est à dire le rapport entre la masse du peptide pur encapsulé et la masse totale de la microcapsule, sera en général comprise entre 0 et 20 %, de préférence entre 2 et 15 %. Pour l'acétate de triptoréline, la charge sera de préférence inférieure ou égale à 10 % et, de façon plus préférentielle, comprise entre 4 et 8 % pour des formes permettant une libération sur une période d'environ 3 mois. Pour l'acétate de lanréotide, la charge sera de préférence comprise entre 10 et 20 %.

Pour des implants, la charge en substance active sera en général comprise entre 0 et 30 % et, de préférence, entre 15 et 25 %.

L'étape d'encapsulation peut être une étape dite de coacervation telle que décrite par exemple dans les brevets américain US 3,773,919 ou européen EP 52 510.

On peut également utiliser un procédé dit de fusion-extrusion tel que décrit dans le brevet européen EP 58 481 ou dans le brevet américain US 5,225,205, les produits obtenus étant ensuite éventuellement broyés selon les méthodes usuelles, pour aboutir à des microparticules.

Par ailleurs, on peut utiliser un principe actif hydrosoluble tel qu'un sel hydrosoluble d'un peptide, par exemple l'acétate. On peut également utiliser un sel insoluble d'une molécule soluble tel qu'un sel d'acide gras d'un peptide, par exemple, un pamoate de peptide tel que décrit dans le brevet britannique GB 2 209 937.

Les compositions obtenues par fusion-extrusion en utilisant les polymères selon l'invention peuvent également se présenter sous forme d'implants et être utilisées en tant que telles.

Ces implants sont de préférence des petits implants (mini-implants ou microimplants) d'un diamètre de l'ordre de 1 mm, par exemple entre 0,8 et 1,2 mm. La longueur de ces implants peut être comprise par exemple entre 10 et 35 mm, par exemple de l'ordre de 25 mm. Ces implants donnent des résultats très intéressants avec de faibles doses de principe actif, par exemple de l'ordre de 3 mg d'acétate de triptoréline par implant. De tels implants peuvent libérer le principe actif pendant une durée pouvant atteindre 3 mois ou plus.

En outre, il est apparu que la configuration du principe actif pouvait également avoir une influence sur la diffusion de ce produit. En particulier, lorsqu'un principe actif peut être obtenu sous une forme cristallisée ou amorphe, le choix de l'une ou l'autre forme n'est pas indifférent.

La demande de brevet EP 709 085 décrit des microcapsules comprenant un polymère et une substance active hydrosoluble et amorphe. Il y est en particulier question de l'importance d'obtenir des particules de substance active de petite taille, et de préférence de taille inférieure à 10 µm. Cette demande ne comporte cependant aucun procédé de préparation desdites particules et aucune mention n'est faite de l'effet de la surface spécifique des particules de principe actif sur le profil de relargage des compositions contenant ces particules. Or la demanderesse utilise déjà depuis 1986 des microcapsules comportant une substance active amorphe, l'acétate de triptoréline vendu sous la dénomination Decapeptyl 3,75 mg, dont la taille des particules est d'environ 8 µm seulement. Mais elle a constaté que la taille des particules n'est pas le seul paramètre déterminant pour favoriser une libération sur une période prolongée pouvant aller jusqu'à plus de trois mois.

La question du caractère amorphe ne se pose en principe pas pour des produits tels que les peptides ou les protéines dont le mode d'obtention, spécialement la lyophilisation, conduit dans la plupart des cas à un produit amorphe, ce qui est le cas pour le Decapeptyl 3,75 mg.

La littérature illustre abondammment ce phénomène et l'on peut citer notamment les articles suivants : Hsu, C.C. et al., Pharmaceutical Research, 12 (1), 69-77 (1995) ou Towns, J.K., Journal of Chromatography, A, 705 (1), 115-27 (1995).

L'invention concerne donc également une composition sous forme de microcapsules ou d'implants comprenant au moins un polymère ou un copolymère biodégradable, de préférence de masse moléculaire élevée, et au moins une substance active hydrosoluble présentant une surface spécifique élevée. En particulier, ladite surface spécifique est supérieure à 2 m²/g, et de préférence supérieure à 3 m²/g. Plus préférentiellement, ladite surface spécifique est supérieure à 5 m²/g ou 10 m²/g. Encore plus préférentiellement, ladite surface spécifique est supérieure à 20 m²/g, et de préférence supérieure à 30 m²/g.

L'invention concerne de préférence les compositions ci-dessus dans lesquelles la substance active hydrosoluble est un peptide ou une protéine.

Elle concerne de même les compositions ci-dessus pour lesquelles la viscosité du polymère ou du copolymère est comprise entre 0,5 et 1,6 dl/g dans CHCl₃, et de préférence comprise entre 0,9 et 1,6 dl/g dans CHCl₃. En particulier, on pourra choisir d'utiliser des polymères ou copolymères de viscosité comprise entre 0,7 et 1,3 dl/g dans CHCl₃, et encore plus préférentiellement des polymères ou copolymères de viscosité comprise entre 0,9 et 1,3 dl/g. Des polymères particulièrement adaptés seront des PLGA. De préférence, lesdits PLGA seront préparés à partir de 40 à 90 % de lactide et de 10 à 60 % de glycolide, et plus préférentiellement à partir de 70 à 80 % de lactide et de 20 à 30 % de glycolide. De préférence, les substances actives hydrosolubles incorporées dans les microcapsules ou implants seront des protéines ou des peptides.

Les compositions comprenant une substance active de surface spécifique élevée seront de préférence telles que la viscosité du polymère ou du copolymère soit comprise entre 0,5 et 1,6 dl/g dans CHCl₃ et que le polymère ou le copolymère présente un caractère hydrophile, l'indice d'acide de ce dernier étant supérieur à 1 meq de KOH par gramme de polymère ou copolymère, et de préférence supérieur à 1,2, plus préférentiellement 1,5 meq voire 2 meq de KOH par gramme de polymère ou copolymère.

L'invention concerne de plus des compositions sous forme de microcapsules ou d'implants comprenant une substance active de surface spécifique élevée caractérisées en ce que le polymère ou copolymère est un PLGA, de préférence un PLGA préparé à partir de 70 à 80 % de lactide et de 20 à 30 % de glycolide, la viscosité dudit PLGA étant comprise entre 0,5 et 1,6 dl/g dans CHCl₃ et la substance active incorporée dans les microcapsules ou implants étant une protéine ou un peptide.

Ces microcapsules ou implants permettent un profil de relargage monophasique dans lequel le pic initial (ou "burst" en anglais) est réduit par rapport à certaines autres préparations utilisant un polymère de poids moléculaire plus faible, de sorte qu'elles permettent de libérer la substance active sur une période prolongée pouvant aller jusqu'à plus de trois mois.

En d'autres termes, la demanderesse a découvert que les propriétés de relargage, notamment le relargage de type monophasique, de compositions sous forme de microcapsules ou d'implants, en particulier de compositions à base de PLGA, et comportant comme principe actif un peptide ou une protéine sont considérablement améliorés si au moins une des caractéristiques suivantes est présente :
a) le polymère ou copolymère est un PLGA qui présente une viscosité dans le chloroforme supérieure à 0,5 dl/g, de préférence supérieure à 0,9 dl/g et inférieure en principe à 1,6 dl/g ;
b) le polymère ou copolymère est un PLGA préparé à partir de 70 à 80 % de lactide et de 20 à 30 % de glycolide ;
c) le polymère ou le copolymère présente un caractère hydrophile, et a de préférence un indice d'acide supérieur à 1 meq KOH, et plus préférentiellement supérieur à 1,2 voire 1,5 meq KOH par gramme de polymère ou de copolymère ;
d) le principe actif, de préférence un peptide ou une protéine, a une surface spécifique élevée et supérieure à 2 m²/g, de préférence supérieure à 10 m²/g, plus préférentiellement supérieure à 20 m²/g et même supérieure à 30 m²/g ;
ces caractéristiques pouvant éventuellement être combinées à l'utilisation d'un L-PLGA au lieu d'un D,L-PLGA.

Dans l'état actuel de ses connaissances, la demanderesse estime que la caractéristique d) est très importante à elle seule et peut être combinée avantageusement aux autres caractéristiques a), b) ou c). En particulier, on pourra combiner la caractéristique d) aux caractéristiques suivantes : a) seule, b) seule, c) seule, a) et b) ensemble, a) et c) ensemble, b) et c) ensemble, ou a), b) et c) ensemble. On combinera plus préférentiellement la caractéristique d) avec au moins la caractéristique c).

Parmi les substances actives utilisables pour les différents aspects de l'invention, on peut notamment citer les protéines et les peptides. Lesdites substances actives pourront être choisies par exemple dans le groupe constitué des substances suivantes : la triptoréline ou un de ses sels, en particulier l'acétate de triptoréline, le lanréotide ou un de ses sels, en particulier l'acétate de lanréotide, l'octréotide ou un de ses sels (tel que décrit par exemple dans le brevet européen EP 29 579), en particulier l'acétate ou le pamoate d'octréotide, un composé ayant une activité LH-RH tel que la triptoréline, la goséréline, la leuproréline, la buséréline ou leurs sels, un antagoniste de LH-RH, un antagoniste de GPIIb/IIIa, un composé ayant une activité similaire à un antagoniste de GPIIb/IIIa, l'érythropoiétine (EPO) ou un de ses analogues, les différents interférons α, l'interféron β ou γ, la somatostatine, un dérivé de la somatostatine tel que décrit dans le brevet européen EP 215 171, un analogue de la somatostatine tel que décrit dans le brevet américain US 5,552,520 (ce brevet comporte lui-même une liste d'autres brevets décrivant des analogues de la somatostatine qui sont incorporés par référence à la présente demande), l'insuline, une hormone de croissance, un facteur libérateur d'hormone de croissance (GRF), un peptide libérateur d'hormone de croissance (GHRP), un facteur de croissance épidermique (EGF), une hormone mélanocyte-stimulante (MSH), une hormone libératrice de thyrotropine (TRH) ou un de ses sels ou dérivés, une hormone stimulant la thyroïde (TSH), une hormone lutéinisante (LH), une hormone stimulant les follicules (FSH), une hormone parathyroïdienne (PTH) ou un de ses dérivés, un hydrochlorure de lysozyme, un peptide relié à l'hormone parathyroïdienne (PTHrp), un fragment de peptide à N terminal (position 1→34) de l'hormone PTH humaine, la vasopressine ou un de ses dérivés, l'oxytocine, la calcitonine, un dérivé de la calcitonine ayant une activité similaire à celle de la calcitonine, un peptide relié au gène de la calcitonine (CGRP), le glucagon, un peptide similaire au glucagon (GLP), la gastrine, un peptide libérateur de gastrine (GRP), la sécrétine, la pancréozymine, la cholécystokinine, l'angiotensine, le lactogène du placenta humain, la gonadotropine chorionique humaine (HCG), l'enképhaline, un dérivé de l'enképhaline, le facteur stimulateur de colonies (CSF), l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombésine ou un de ses dérivés tels que décrits dans le brevet américain US 5,552,520 (ce brevet comporte lui-même une liste d'autres brevets décrivant des dérivés de la bombésine qui sont incorporés par référence à la présente demande), la prolactine, la neurotensine, la dynorphine, la caeruléine, la substance P, l'urokinase, l'asparaginase, la bradykinine, la kallikréine, la facteur de croissance nerveuse, un facteur de coagulation sanguine, la polymixine B, la colistine, la gramicidine, la bacitracine, un peptide stimulant la synthèse protéique, un antagoniste de l'endothéline ou un de ses sels ou dérivés, un polypeptide intestinal vasoactif (VIP), l'hormone adrénocorticotropique (ACTH) ou un de ses fragments, un facteur de croissance dérivé des plaquettes (PDGF), une protéine morphogénétique osseuse (BMP), un polypeptide activant l'adénylatecyclase pituitaire (PACAP), le neuropeptide Y (NPY), le peptide YY (PYY), un polypeptide inhibiteur gastrique (GIP), et des polynucléotides, notamment des ARN double brin (ARNdb) tels ceux décrits dans la demande de brevet EP 0 300 680 ou le brevet français No. 2 622 586.

Par ARNdb, on entend de préférence l'acide polyadénylique complexé avec l'acide polyuridylique, aussi appelé poly(A)-poly(U) ou Poly-adenur®. D'autres ARNdb peuvent être utilisés pour l'invention, notamment un complexe de l'acide polyinosinique avec l'acide polycytidylique, également connu sous le nom de poly(I)-poly(C), ainsi que ces mêmes complexes modifiés par introduction d'acide uridylique dans la chaîne de l'acide polycytidylique, tel le produit Ampligen® de la société HEMISPHERx (pour une description de ces produits, se référer notamment à la demande de brevet européen EP 0 300 680). L'ARNdb utilisé peut être par exemple un mélange d'ARNdb tels que défini ci-dessus. De préférence, les ARNdb sont préparés selon le procédé décrit dans le brevet français No. 2 622 586.

Une surface spécifique élevée peut être obtenue pour les substances actives précitées dès lors qu'elles sont hydrosolubles ou rendues hydrosolubles, par exemple par salification ou par greffe sur leur structure d'une chaîne hydrosoluble. Ceci est en particulier valable pour les peptides et les protéines précitées. Toute autre substance active hydrosoluble, ou un de ses sels ou précurseurs, et en particulier les sels obtenus par salification avec l'acide acétique, pourra également être utilisée par l'homme du métier pour cet aspect de l'invention s'il le juge utile.

Selon un des aspects préférés de l'invention, le peptide ou la protéine de surface spécifique élevée sont choisis dans un groupe composé de l'acétate de triptoréline, l'acétate de lanréotide ou l'acétate d'octréotide.

Par peptide et/ou protéine, on entend dans la présente demande aussi bien le peptide et/ou la protéine eux-mêmes que des fragments, sels ou dérivés pharmacologiquement actifs de ces peptides ou protéines.

La substance active hydrosoluble telle qu'utilisée pour fabriquer des microcapsules ou des implants selon l'invention, et en particulier l'acétate de triptoréline, l'acétate de lanréotide, l'acétate d'octréotide, la goséréline, la leuproréline, la buséréline ou leurs sels, est obtenue de préférence par un procédé comportant principalement deux étapes :
- une étape de lyophilisation comprenant une trempe rapide d'une solution diluée de la substance hydrosoluble dans un milieu de température inférieure à -50 °C, et de préférence inférieure à -70 °C ;
- éventuellement une étape de broyage ; de préférence, cette étape comprendra un broyage ultrasonique.

Par solution diluée de la substance active, on entend une solution ayant une concentration de ladite substance active inférieure à la moitié de la concentration de saturation, et de préférence inférieure à un quart de ladite concentration de saturation lorsque celle-ci est au moins égale à 200 g/l. Ce procédé permet d'obtenir une substance active présentant une surface spécifique élevée.

Par trempe rapide, il faut entendre une mise en contact avec un milieu à basse température provoquant une congélation instantanée de la solution de substance hydrosoluble.

Pour la lyophilisation, on pourra par exemple congeler la solution dans un plateau baignant dans un bac d'azote liquide, avant de procéder à la lyophilisation proprement dite.

De préférence, afin d'obtenir une surface spécifique maximale, la trempe rapide de la solution sera précédée d'une micronisation de la solution de substance active. Lorsque la solution de substance active est préalablement micronisée, la température du milieu à basse température pourra être inférieure à -50 °C seulement.

Par exemple, pour obtenir une surface spécifique très élevée, on pourra choisir d'atomiser la solution en la pulvérisant à travers un atomiseur sur une plaque métallique à très basse température. La température de la plaque sera de préférence inférieure à -50°C, et plus préférentiellement inférieure à -70 °C voire -80 °C ou -120 °C. Cette température pourra être atteinte par exemple en faisant tremper une plaque métallique dans un milieu à très basse température comme par exemple de l'azote liquide. Selon une variante préférée de l'invention, la plaque métallique est creuse et la solution est pulvérisée à l'aide d'un atomiseur à l'intérieur de ladite plaque.

D'autres techniques de congélation sont envisageables, par exemple l'atomisation de la solution de substance active dans un bain de non-solvant de ladite substance active préalablement réfrigéré. Comme non-solvant, on préférera un gaz liquéfié comme par exemple l'azote liquide.

Une autre possibilité est de congeler la solution de substance active sur un plateau tournant réfrigéré ("drum-freezing"). Comme indiqué précédemment, cette congélation sera de préférence précédée d'une micronisation de la solution de substance active.

Lorsque le procédé de congélation dans un plateau est appliqué à une substance active pour préparer des microcapsules ou des implants à libération prolongée selon l'invention, la surface spécifique de la substance active, après lyophilisation mais avant broyage, sera de préférence supérieure à 2 m²/g. De façon plus préférentielle, la surface spécifique de la substance active sera supérieure à 3 m²/g, voire 5 m²/g.

Si une surface spécifique supérieure à 10 m²/g est nécessaire, il sera préférable de recourir au procédé incluant une étape de micronisation. De préférence, la surface spécifique obtenue pour la substance active après lyophilisation sera supérieure à 15 m²/g. Encore plus préférentiellement, cette surface spécifique sera supérieure à 20 m²/g, voire 30 m²/g.

Pour faire varier les surfaces spécifiques obtenues, on pourra faire varier les conditions de congélation de la solution de substance active en jouant sur différents paramètres tels que par exemple la vitesse de congélation ou la concentration de la solution.

La surface spécifique de la substance active est un facteur favorable pour obtenir une libération sur une période prolongée, en particulier dans le cas des microcapsules. En effet, comme déjà évoqué, des particules d'une substance active de même taille mais de surfaces spécifiques différentes donneront avec le même excipient polymère des résultats tout à fait différents.

L'invention a donc également pour objet les procédés tel que décrits ci-dessus appliqués à une substance hydrosoluble biologiquement active. Elle concerne aussi la substance hydrosoluble biologiquement active telle qu'obtenue par ces procédés, laquelle présente une surface spécifique élevée.

En particulier, l'invention concerne de l'acétate de triptoréline, de l'acétate de lanréotide ou de l'acétate d'octréotide tel qu'obtenu par les procédés décrits précédemment, ou un ARN double brin, de préférence un complexe d'acide polyadénylique avec de l'acide polyuridylique, tel qu'obtenu par ces procédés.

Comme indiqué ci-dessus, les compositions selon l'invention trouvent leur usage de préférence dans le domaine pharmaceutique. Les compositions pharmaceutiques peuvent être administrées à un patient par différentes voies ; cependant, la voie préférée est la voie injectable sous-cutanée ou intramusculaire. Les microcapsules selon l'invention peuvent d'abord être suspendues dans un véhicule approprié destiné à l'injection, comme une solution aqueuse de chlorure de sodium ou une solution aqueuse de mannitol.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES :

Pour tous ces exemples, les viscosités inhérentes (IV) ont été mesurées selon les méthodes classiques de mesure du temps d'écoulement telles que décrites par exemple dans "Pharmacopée Européenne", 1997, pages 17-18 (méthode au tube capillaire). Sauf mention contraire, ces viscosités ont été mesurées dans le chloroforme à une concentration de 0,1 % à 25 °C, ou dans l'hexafluoroisopropanol à une concentration de 0,5 % à 30 °C. La surface spécifique de la substance active, lorsqu'elle a été mesurée, a été déterminée par la méthode dite méthode B.E.T. (absorption d'une monocouche d'azote sur la substance active), méthode bien connue de l'homme du métier.

Pour les exemples suivants, on appellera peptide "modifié" un peptide ayant subi le procédé de lyophilisation selon l'invention, par opposition au peptide "non modifié", qui est lyophilisé de façon classique (sans trempe brutale à basse température).

### Exemple 1 :

16,620 g d'acétate de triptoréline "non modifié" sont dissous dans 554 ml d'eau. La solution est congelée dans un plateau baignant dans un bac d'azote liquide, puis lyophilisée.

15,18 g d'acétate de triptoréline "modifié" sont ainsi obtenus avec un rendement de 91,34 %. Ce composé présente une surface spécifique de 4,7 m²/g contre 0,8 m²/g avant lyophilisation.

On réalise ensuite le broyage aux ultrasons de l'acétate de triptoréline : 15 minutes sont suffisantes pour obtenir des particules inférieures à 10 µm pour le peptide modifié (alors que 30 minutes sont nécessaires pour obtenir une telle granulométrie avec le peptide non modifié).

L'étape d'encapsulation est ensuite réalisée selon la méthode de coacervation telle que décrite dans les brevets européen EP 52 510 et américain US 3,773,919; au départ de 3,378 g de cet acétate de triptoréline modifié et broyé et d'une solution à 7,30 % de D,L-PLGA (D,L-PLGA composé de 75 % de DL-lactide et 25 % de glycolide, viscosité inhérente dans le chloroforme = 0,70 dl/g, indice d'acide = 1,61 meq KOH / g) dans du dichlorométhane. 390 ml d'huile de silicone ont été additionnés pour former des microcapsules par le procédé de coacervation. Ces microcapsules sont récupérées après immersion dans un bain d'heptane (221) et filtration sur membrane 10 µm.

### Exemple 2 :

0,338 g d'acétate de triptoréline non modifié, de granulométrie égale à 8 µm après broyage aux ultrasons pendant 30 minutes, ont été additionnés sous agitation à une solution à 7,30 % de D,L-PLGA dans du dichlorométhane (PLGA équivalent à celui décrit dans l'exemple 1). 40 ml d'huile de silicone ont été additionnés pour former des microcapsules qui sont par la suite précipitées dans un bain d'heptane (2 1) puis filtrées sur membrane 10 µm.

### Exemples 3 à 6 :

0,338 g d'acétate de triptoréline modifié selon les conditions décrites dans le Tableau No. 1 ci-dessous, ont été additionnés, après broyage aux ultrasons, à une solution à 7,30 % d'un mélange 33,3 % / 33,3 % / 33,3 % de trois D,L-PLGA (aux caractéristiques décrites dans le Tableau No. 2 ci-dessous) dans du dichlorométhane. 40 ml d'huile de silicone ont été additionnés pour former des microcapsules qui sont par la suite précipitées dans un bain d'heptane (2 1) puis filtrées sur membrane 10 µm.

**Tableau No. 1**

| **Exemple** | **Concentration g/l** | **Quantité acétate de triptoréline (g)** | **Quantité d'eau (ml)** | **Surface spécifique m²/g** |
|---|---|---|---|---|
| 3 | 200 | 3 | 15 | 4,4 |
| 4 | 150 | 3 | 20 | 4,7 |
| 5 | 100 | 3 | 30 | 4,8 |
| 6 | 50 | 3 | 60 | 7,3 |

La surface spécifique de l'acétate de triptoréline de départ (non modifié) est de 0,8 m²/g.

Les caractéristiques physico-chimiques des trois polymères mélangés sont réunies dans le Tableau No. 2 ci-après :

**Tableau No. 2**

| **Caractéristiques** | **PLGA No. 1** | **PLGA No. 2** | **PLGA No. 3** |
|---|---|---|---|
| Ratio lactide / glycolide | D,L-PLGA 50:50 | D,L-PLGA 75:25 | D,L-PLGA 75:25 |
| Viscosité inhérente dans CHCl₃ (dl/g) | 0,47 | 0,61 | 0,70 |
| Indice d'acide (meq KOH/g) | 2,68 | 2,08 | 1,61 |

### Exemple 7 :

22,560 g d'acétate de lanréotide non modifié sont dissous dans 752 ml d'eau. La solution est congelée dans un plateau baignant dans un bac d'azote liquide, puis lyophilisée. 21,75 g d'acétate de lanréotide modifié, de surface spécifique égale à 4,4 m²/g, sont obtenus avec un rendement de 96,41 %.

L'étape d'encapsulation est ensuite réalisée selon la méthode de coacervation telle que décrite dans les brevets européen EP 52 510 et américain US 3,773,919 ; au départ de 7,5 g de cet acétate de triptoréline modifié et broyé et d'une solution à 3,7 % de D,L-PLGA (D,L-PLGA composé de 50 % de DL-lactide et 50 % de glycolide, viscosité inhérente dans HFIP = 0,55 dl/g) dans du dichlorométhane. 650 ml d'huile de silicone ont été additionnés pour former des microcapsules par le procédé de coacervation. Ces microcapsules sont récupérées après immersion dans un bain d'heptane (30 1) et filtration sur membrane 10 µm.

### Exemples 8 et 9 :

Des microcapsules d'acétate de triptoréline ont été fabriquées avec D,L-PLGA (D,L-PLGA composé de 75 % de DL-lactide et 25 % de glycolide) de différentes masses moléculaires moyennes en poids (Mw). Les fabrications ont été effectuées selon le procédé décrit dans l'exemple 1 avec un acétate de triptoréline de surface spécifique égal à 4,7 m²/g.

Les paramètres physico chimiques des exemples 8 et 9 sont réunis dans le tableau ci-après :

| **Exemple** | **Mw THF** | **IV CHCl₃ (dl/g)** | **Indice d'acide (meq KOH/g)** |
|---|---|---|---|
| 8 | 58400 | 0,61 | 2,08 |
| 9 | 132650 | 0,93 | 1,31 |

### Exemple 10 :

Des microcapsules ont été fabriquées selon le procédé décrit dans l'exemple 1 avec D,L-PLGA (D,L-PLGA composé de 75 % de DL-lactide et 25 % de glycolide ; masse moléculaire déterminée dans le THF : 80100 ; viscosité dans le chloroforme : 0,75 dl/g, indice
d'acide = 0,40 meq KOH / g) à tendance hydrophobe.

### Exemple 11 :

Des microcapsules ont été fabriquées selon le procédé décrit dans l'exemple 1, à partir d'un L-PLGA (L-PLGA composé de 75 % de L-lactide et 25 % de glycolide ; masse moléculaire dans le THF : 99260 ; viscosité dans le chloroforme : 0,78 dl/g, indice d'acide = 1,80 meq KOH / g) à tendance cristalline.

### Exemple 12 :

A quatre parties, en poids, de poudre de D,L-PLGA (PLGA composé de 75 % de lactide et 25 % de glycolide ; masse moléculaire déterminée dans le THF : 103810 ; viscosité inhérente dans le chloroforme : 0,82 dl/g) on ajoute une partie, en poids, d'acétate de triptoréline.

On détruit les grumeaux par un tamisage sur une maille 400 µm, on mélange 20 minutes à 42 tours par minute et on extrude le mélange à 120° C avec une extrudeuse à vis, à travers une filière de 1 mm d'ouverture. On refroidit ensuite à l'air et on calibre par étirage (étireuse) à un diamètre final de 0,85 mm.

La richesse du mélange par unité de longueur (mm) est déterminée et on dose les microimplants à 3 mg de triptoréline en coupant les tiges d'extrudat à des longueurs calculées (ici, 24 mm). Enfin, le poids de chaque microimplant est contrôlé.

### Exemples 13 et 14 :

Le même protocole est utilisé pour ces deux exemples :

5 g d'acétate de lanréotide sont dissous dans de l'eau pour donner la concentration choisie à la solution (par exemple, pour obtenir la concentration de 30 g/l, on ajoute 167 ml d'eau stérile). Cette solution est atomisée à l'aide d'un pulvérisateur de 500 ml, dont le jet est réglé de façon à obtenir les goutelettes les plus fines possibles. Les gouttelettes obtenues sont projetées dans un plateau dont le fond trempe dans l'azote liquide. Deux sondes de température sont préalablement introduites dans le plateau afin de suivre l'évolution de la température du produit.

Une fois le produit congelé, le plateau est introduit dans un lyophilisateur dont la plaque est à environ -54 °C.

On laisse s'équilibrer la température des produits et de la plaque pendant 1 heure. Puis on passe à la phase de sublimation (la température de la plaque est fixée à 20 °C et la pression dans la cuve à 100 µbar). Cette phase dure environ 30 heures. La température finale moyenne du produit est de 13 °C. La dessiccation secondaire qui suit (pression dans la cuve 50 µbar) dure environ 24 heures. La température finale moyenne du produit est de 20 °C.

Les caractéristiques des réactifs engagés et des produits obtenus sont résumés dans le tableau ci-après :

| Caractéristiques | Exemple 13 | Exemple 14 |
|---|---|---|
| Masse d'acétate de lanréotide engagée (g) | 5,00 | 5,00 |
| Concentration de la solution (g/l) | 30 | 10 |
| Masse d'acétate de lanréotide récupérée | 4,54 | 4,10 |
| Surface spécifique obtenue (m²/g) | 36 | 43 |

L'acétate de lanréotide de surface spécifique 43 m²/g obtenu précédemment (exemple 14) est incorporé dans des microcapsules selon le procédé suivant :

0,782 g d'acétate de lanréotide sont pesés dans un tube en verre. 15 ml de dichlorométhane sont ajoutés au sel de peptide. Le broyage du peptide est effectué aux ultrasons à l'aide d'un générateur à ultrasons équipé d'un amplificateur et d'une sonde à extrémité plate ou plongeante (fréquence = 50 Hz, puissance 250 W ; broyage durant environ 15 min).

L'étape d'encapsulation est ensuite réalisée selon la méthode de coacervation telle que décrite dans les brevets européen EP 52 510 et américain US 3,773,919 en utilisant les 0,782 g d'acétate de lanréotide broyés et une solution de 4 g de D,L-PLGA 50:50 (IV = 0,48 dl/g dans CHCl₃) dans 35 ml de dichlorométhane. 34,2 ml d'huile de silicone ont été additionnés pour former des microcapsules par le procédé de coacervation. Ces microcapsules sont récupérées après immersion dans un bain d'heptane (2,5 1) et filtration sur membrane 10 µm.

Les microcapsules obtenues peuvent ensuite être séchées sous vide, réparties en flacons, et lyophilisées avec des excipients (par exemple du ballast ou un surfactant) afin d'être stockées dans de bonnes conditions et faciliter la mise en suspension des microcapsules.

### Exemples 15 et 16 :

Un protocole analogue à celui de l'exemple 1 est utilisé pour ces deux exemples. Le peptide utilisé est le même que celui de ces exemples. Les caractéristiques en termes de PLGA utilisé, de quantité de peptide engagé (pour ces exemples, l'acétate de triptoréline modifié) et de paramètres de préparation des microcapsules sont reportées dans le tableau ci-après :

| Caractéristiques | Exemple 15 | Exemple 16 |
|---|---|---|
| Masse d'acétate de triptonéline modifié engagée (g) | 2,58 | 2,58 |
| Quantité de polymère engagée (g) | 30 | 30 |
| Quantité d'huile de silicone engagée (ml) | 600 | 650 |
| Quantité de dichlorométhane engagée (ml) | 812 | 812 |
| Proportion lactide / glycolide | 75 / 25 | 75 / 25 |
| Viscosité du PLGA dans CHCl₃ (dl/g) | 0,96 | 0,96 |
| Indice d'acide mesuré | 1,22 | 1,12 |

### Etude des profils de libération de microcapsules selon l'invention :

Afin d'illustrer l'intérêt de microcapsules selon l'invention, l'étude de leurs profils de libération *in vitro* a été réalisée.

Pour chacun des exemples 1 à 11 et 15 à 16, on mesure la libération de trois prises d'essai d'environ 25 mg de microcapsules (environ 20 mg pour l'exemple 7), placées dans 4 ml de solution de chlorure de sodium à 0,9 %. On extrait après 1 heure, 1 jour et 4 jours de libération dans la solution maintenue à 37° C.

On dose la triptoréline par chromatographie liquide de haute performance (HPLC) par rapport à une gamme d'étalonnage en mode gradient dans le système acide trifluoroacétique (TFA).

Pour obtenir la gamme d'étalonnage standard pour la triptoréline, on prépare une solution T₁ de la façon suivante : une prise d'essai voisine de 7,5 mg d'acétate de triptoréline de référence est placée dans une fiole de 50 ml ; on complète à 50 ml avec une solution d'acide acétique à 0,1 %. A partir de la solution T₁, on prépare les solutions T₂ et T₃ en procédant comme suit : pour T₂, on prélève 10 ml de solution T₁ et on complète à 20 ml avec la solution d'acide acétique à 0,1 %. Pour la solution T₃, on prélève 1 ml de solution T₁ et on complète à 50 ml avec la solution d'acide acétique à 0,1 %.

Le lanréotide est dosé de façon analogue par HPLC. Pour obtenir la gamme d'étalonnage standard pour le lanréotide, on prépare une solution T'₁ de la façon suivante : une prise d'essai voisine de 16,5 mg d'acétate de triptoréline de référence est placée dans une fiole de 50 ml ; on complète à 50 ml avec une solution d'acide acétique à 0,1 %. A partir de la solution T'₁, on prépare les solutions T'₂, T'₃, T'₄ et T'₅ en procédant comme suit : pour T'₂, on prélève 10 ml de solution T'₁ et on complète à 25 ml avec la solution d'acide acétique à 0,1 %. Pour la solution T'₃, on prélève 5 ml de solution T'₁ et on complète à 25 ml avec la solution d'acide acétique à 0,1 %. La solution T'₄ est obtenue par dilution de 2 ml de solution T'₁ dans une solution d'acide acétique à 0,1 % pour obtenir un volume total de 25 ml, et la solution T'₅ par dilution de 1 ml de solution T'₁ dans une solution d'acide acétique à 0,1 % pour obtenir un volume total de 25 ml.

La quantité d'acétate de triptoréline ou d'acétate de lanréotide libérée est déterminée en pourcentage par rapport à la quantité d'acétate de triptoréline ou d'acétate de lanréotide initialement présente (100 %), qui sert de référence.

Les résultats des tests *in vitro* sont résumés par le tableau ci-dessous :

| **Exemples** | **Taux de libération cumulé (%)** | | |
|---|---|---|---|
| | **1 heure** | **1 jour** | **4 jours** |
| 1 | 8 | 16 | 27 |
| 2 | 9 | 47 | 57 |
| 3 | 10 | 45 | 67 |
| 4 | 10 | 37 | 65 |
| 5 | 9 | 41 | 66 |
| 6 | 8 | 30 | 55 |
| 7 | 3 | 14,5 | 28,3 |
| 8 | 11 | 40 | 67 |
| 9 | 2 | 4 | 6 |
| 10 | 5 | 12 | 14 |
| 11 | 1 | 2 | 2 |
| 15 | 2 | 4 | 13 |
| 16 | 2 | 5 | 10 |

Les résultats des tests *in vivo* sont parfaitement bien corrélés à ceux des tests *in vitro.* A titre d'exemple, des microcapsules de l'exemple 1, à la dose de 1,2 mg/kg, ont été injectées par voie intramusculaire à des rats. Un dosage plasmatique a révélé que le taux de triptoréline restait constamment supérieur à 0,1 ng/ml sur une période de plus de 90 jours. Des mêmes études menées sur des microcapsules de l'exemple 2 ont montré que le taux de testostérone restait constamment inférieur à 1 ng/ml sur une période de plus de 90 jours. Par ailleurs, des microcapsules de l'exemple 9 ont aussi été injectées par voie intramusculaire à des rats à une dose de 1,2 mg/kg et un dosage plasmatique a montré que le taux de triptoréline restait constamment supérieur à 0,1 ng/ml sur une période de plus de 90 jours.

Les microimplants de l'exemple 12 ont été testés *in vivo* de la façon suivante : une dose totale de 3 mg de triptoréline a été injectée en intramusculaire sur 6 chiens Beagles (poids d'environ 12 kg) dans un muscle de la patte arrière de chacun des animaux. Un dosage plasmatique a révélé que le taux de triptoréline restait constamment supérieur à 0,1 ng/ml sur une période de plus de 90 jours.

## Revendications

1. Composition sous forme de microcapsules ou d'implants comprenant un excipient ou un mélange d'excipients polymères ou copolymères biodégradables et une substance active ou un mélange de substances actives, lesdites microcapsules ou lesdits implants pouvant libérer la substance active ou le mélange de substances actives sur une période prolongée pouvant aller jusqu'à trois mois ou plus selon un profil essentiellement monophasique, ladite composition étant **caractérisée en ce que** :
lorsque la composition est sous forme de microcapsules, les excipients comprennent des copolymères dont la viscosité est comprise entre 0,9 dl/g et 1,6 dl/g dans CHCI₃ et le procédé de préparation desdites microcapsules ne comporte pas d'étape de fusion desdites microcapsules
- ou bien, lorsque la composition est sous forme d'implants, la viscosité desdits polymères ou copolymères est comprise entre 0,5 dl/g et 1,6 dl/g dans CHCl₃.

2. Composition sous forme de microcapsules ou d'implants selon la revendication 1, **caractérisée en ce que** l'excipient polymère ou copolymère biodégradable possède une viscosité inhérente au moins égale à 0,9 dl/g dans CHCl₃.

3. Composition sous forme de microcapsules ou d'implants selon l'une des revendications 1 ou 2, **caractérisée en ce que** le polymère biodégradable est un copolymère de lactide et de glycolide (PLGA).

4. Composition selon la revendication 3, **caractérisée en ce que** l'indice d'acide du polymère ou copolymère est supérieur à 1 meq de KOH par gramme de polymère ou copolymère, et de préférence supérieur à 1,2 ou 1,5 meq de KOH par gramme de polymère ou copolymère.

5. Composition sous forme de microcapsules ou d'implants selon la revendication 3 ou 4, **caractérisée en ce que** le PLGA est préparé à partir de 70 à 80 % de lactide et de 20 à 30 % de glycolide.

6. Composition sous forme de microcapsules ou d'implants selon l'une des revendications 1 à 5, **caractérisée en ce que** le polymère biodégradable est un copolymère de L-lactide et de glycolide.

7. Composition sous forme de microcapsules ou d'implants comprenant au moins un excipient ou un mélange d'excipients polymères ou copolymères biodégradables et au moins une substance active hydrosoluble présentant une surface spécifique élevée.

8. Composition sous forme de microcapsules ou d'implants selon la revendication 7, **caractérisée en ce que** la surface spécifique de la substance active est supérieure à 2 m²/g, et de préférence supérieure à 3 m²/g.

9. Composition sous forme de microcapsules ou d'implants selon la revendication 8, **caractérisée en ce que** la surface spécifique de la substance active est supérieure à 5 m²/g, et de préférence supérieure à 10 m²/g.

10. Composition sous forme de microcapsules ou d'implants selon la revendication 9, **caractérisée en ce que** la surface spécifique de la substance active est supérieure à 20 m²/g, et de préférence supérieure à 30 m²/g.

11. Composition sous forme de microcapsules ou d'implants selon l'une quelconque des revendications 7 à 10, **caractérisée en ce que** la viscosité du polymère ou du copolymère est comprise entre 0,5 et 1,6 dl/g dans CHCl₃, et de préférence comprise entre 0,9 et 1,6 dl/g dans CHCl₃.

12. Composition sous forme de microcapsules ou d'implants selon l'une quelconque des revendications 7 à 11, **caractérisée en ce que** le polymère ou le copolymère présente un caractère hydrophile, l'indice d'acide de ce dernier étant supérieur à 1 meq de KOH par gramme de polymère ou copolymère, et de préférence supérieur à 1,2 ou 1,5 meq de KOH par gramme de polymère ou copolymère.

13. Composition sous forme de microcapsules ou d'implants selon l'une quelconque des revendications 7 à 12, **caractérisée en ce que** le polymère ou copolymère est un PLGA, de préférence un PLGA préparé à partir de 70 à 80 % de lactide et de 20 à 30 % de glycolide.

14. Composition sous forme de microcapsules ou d'implants selon l'une quelconque des revendications 1 à 13, **caractérisée en ce que** la substance active est une protéine ou un peptide.

15. Composition sous forme de microcapsules ou d'implants selon l'une des revendications précédentes, **caractérisée en ce que** la substance active est choisie dans le groupe constitué des substances suivantes : la triptoréline ou un de ses sels, en particulier l'acétate de triptoréline, le lanréotide ou un de ses sels, en particulier l'acétate de lanréotide, l'octréotide ou un de ses sels, en particulier l'acétate ou le pamoate d'octréotide, un composé ayant une activité LH-RH tel que la triptoréline, la goséréline, la leuproréline, la buséréline ou leurs sels, un antagoniste de LH-RH, un antagoniste de GPIIb/IIIa, un composé ayant une activité similaire à un antagoniste de GPIIb/IIIa, l'érythropoiétine (EPO) ou un de ses analogues, les différents interférons α, l'interféron β ou γ, la somatostatine, un dérivé de la somatostatine, un analogue de la somatostatine, l'insuline, une hormone de croissance, un facteur libérateur d'hormone de croissance (GRF), un peptide libérateur d'hormone de croissance (GHRP), un facteur de croissance épidermique (EGF), une hormone mélanocyte-stimulante (MSH), une hormone libératrice de thyrotropine (TRH) ou un de ses sels ou dérivés, une hormone stimulant la thyroïde (TSH), une hormone lutéinisante (LH), une hormone stimulant les follicules (FSH), une hormone parathyroïdienne (PTH) ou un de ses dérivés, un hydrochlorure de lysozyme, un peptide relié à l'hormone parathyroïdienne (PTHrp), un fragment de peptide à N terminal (position 1→34) de l'hormone PTH humaine, la vasopressine ou un de ses dérivés, l'oxytocine, la calcitonine, un dérivé de la calcitonine ayant une activité similaire à celle de la calcitonine, un peptide relié au gène de la calcitonine (CGRP), le glucagon, un peptide similaire au glucagon (GLP), la gastrine, un peptide libérateur de gastrine (GRP), la sécrétine, la pancréozymine, la cholécystokinine, l'angiotensine, le lactogène du placenta humain, la gonadotropine chorionique humaine (HCG), l'enképhaline, un dérivé de l'enképhaline, le facteur stimulateur de colonies (CSF), l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombésine ou un de ses dérivés, la prolactine, la neurotensine, la dynorphine, la caeruléine, la substance P, l'urokinase, l'asparaginase, la bradykinine, la kallikréine, le facteur de croissance nerveuse, un facteur de coagulation sanguine, la polymixine B, la colistine, la gramicidine, la bacitracine, un peptide stimulant la synthèse protéique, un antagoniste de l'endothéline ou un de ses sels ou dérivés, un polypeptide intestinal vasoactif (VIP), l'hormone adrénocorticotropique (ACTH) ou un de ses fragments, un facteur de croissance dérivé des plaquettes (PDGF), une protéine morphogénétique osseuse (BMP), un polypeptide activant l'adénylatecyclase pituitaire (PACAP), le neuropeptide Y (NPY), le peptide YY (PYY), un polypeptide inhibiteur gastrique (GIP), et des polynucléotides, notamment des ARN double brin.

16. Composition sous forme de microcapsules ou d'implants selon l'une des revendications 1 à 15, **caractérisée en ce que** la substance active est choisie parmi un groupe composé de l'acétate de triptoréline, l'acétate de lanréotide ou l'acétate d'octréotide.

17. Procédé de préparation d'une composition selon l'une des revendications précédentes comprenant la préparation d'une substance hydrosoluble présentant une surface spécifique élevée, comprenant les étapes suivantes :
- une étape de lyophilisation de la substance hydrosoluble comprenant une trempe rapide d'une solution diluée de ladite substance hydrosoluble dans un milieu de température inférieure à -50 °C, et de préférence inférieure à - 70 °C ;
- éventuellement une étape de broyage, laquelle comprend de préférence un broyage ultrasonique.

18. Procédé selon la revendication 17, **caractérisé en ce que** la solution diluée est une solution à une concentration inférieure à la moitié de la concentration de saturation.

19. Procédé selon la revendication 17 ou 18, **caractérisé en ce que** la substance hydrosoluble présente une concentration de saturation d'au moins 200 g/l et que la solution diluée est une solution à une concentration inférieure à un quart de la concentration de saturation.

20. Procédé selon l'une des revendications 17 à 19, **caractérisé en ce que** la trempe rapide est réalisée après une étape de micronisation de la solution de substance active, ladite étape de micronisation consistant de préférence en le passage de la solution de substance active dans un atomiseur.

21. Composition selon la revendication 1-16 comprenant une substance active, de préférence une protéine ou un peptide, telle qu'obtenue par un procédé selon l'une des revendications 17 à 20.

22. Composition selon la revendication 21, **caractérisée en ce que** la substance active est choisie dans le groupe constitué des substances suivantes : la triptoréline ou un de ses sels, en particulier l'acétate de triptoréline, le lanréotide ou un de ses sels, en particulier l'acétate de lanréotide, l'octréotide ou un de ses sels, en particulier l'acétate d'octréotide, un composé ayant une activité LH-RH tel que la triptoréline, la goséréline, la leuproréline, la buséréline ou leurs sels, un antagoniste de LH-RH, un antagoniste de GPIIb/IIIa, un composé ayant une activité similaire à un antagoniste de GPIIb/IIIa, l'érythropoiétine (EPO) ou un de ses analogues, les différents interférons α, l'interféron β ou γ, la somatostatine, un dérivé de la somatostatine, un analogue de la somatostatine, l'insuline, une hormone de croissance, un facteur libérateur d'hormone de croissance (GRF), un peptide libérateur d'hormone de croissance (GHRP), un facteur de croissance épidermique (EGF), une hormone mélanocyte-stimulante (MSH), une hormone libératrice de thyrotropine (TRH) ou un de ses sels ou dérivés, une hormone stimulant la thyroïde (TSH), une hormone lutéinisante (LH), une hormone stimulant les follicules (FSH), une hormone parathyroïdienne (PTH) ou un de ses dérivés, un peptide relié à l'hormone parathyroïdienne (PTHrp), un fragment de peptide à N terminal (position 1→34) de l'hormone PTH humaine, la vasopressine ou un de ses dérivés, l'oxytocine, la calcitonine, un dérivé de la calcitonine ayant une activité similaire à celle de la calcitonine, un peptide relié au gène de la calcitonine (CGRP), le glucagon, un peptide similaire au glucagon (GLP), la gastrine, un peptide libérateur de gastrine (GRP), la sécrétine, la pancréozymine, la cholécystokinine, l'angiotensine, le lactogène du placenta humain, la gonadotropine chorionique humaine (HCG), l'enképhaline, un dérivé de l'enképhaline, le facteur stimulateur de colonies (CSF), l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombésine ou un de ses dérivés, la prolactine, la neurotensine, la dynorphine, la caeruléine, la substance P, l'urokinase, l'asparaginase, la bradykinine, la kallikréine, le facteur de croissance nerveuse, un facteur de coagulation sanguine, la polymixine B, la colistine, la gramicidine, la bacitracine, un peptide stimulant la synthèse protéique, un antagoniste de l'endothéline ou un de ses sels ou dérivés, un polypeptide intestinal vasoactif (VIP), l'hormone adrénocorticotropique (ACTH) ou un de ses fragments, un facteur de croissance dérivé des plaquettes (PDGF), une protéine morphogénétique osseuse (BMP), un polypeptide activant l'adénylatecyclase pituitaire (PACAP), le neuropeptide Y (NPY), le peptide YY (PYY), un polypeptide inhibiteur gastrique (GIP), et des polynucléotides, notamment des ARN double brin.

23. Composition selon la revendication 21 comprenant l' acétate de triptoréline, acétate de lanréotide ou acétate d'octréotide tel qu'obtenu par un procédé selon l'une des revendications comme principe actif.

24. Composition selon la revendication 21 comprenant l'ARN double brin, de préférence un complexe d'acide polyadénylique avec de l'acide polyuridylique, tel qu'obtenu par un procédé selon l'une des revendications comme principe actif.

## Claims

1. Composition in the form of microcapsules or implants comprising a biodegradable polymeric or copolymeric excipient or a mixture of such excipients and an active substance or a mixture of active substances, it being possible for said microcapsules or said implants to release the active substance or the mixture of active substances over a prolonged period of up to three months or more according to an essentially monophase release profile, said composition being **characterized in that** :
- when the composition is in the form of microcapsules, the excipients contain copolymers the viscosity of which is comprised between 0.9 dl/g and 1.6 dl/g in CHCl₃ and the preparation process for said microcapsules does not comprise any stage of fusion of said microcapsules,
- or, when the composition is in the form of implants the viscosity of said polymers or copolymers is comprised between 0.5 dl/g and 1.6 dl/g in CHCl₃.

2. Composition in the form of microcapsules or implants according to claim 1, **characterized in that** the biodegradable polymeric or copolymeric excipient has an inherent viscosity at least equal to 0.9 dl/g in CHCl₃.

3. Composition in the form of microcapsules or implants according to one of claims 1 or 2, **characterized in that** the biodegradable polymer is a copolymer of lactide and glycolide (PLGA).

4. Composition according to claim 3, **characterized in that** the acid number of the polymer or copolymer is greater than 1 meq of KOH per gram of polymer or copolymer , and preferably greater than 1.2 or 1.5 meq of KOH per gram of polymer or copolymer.

5. Composition in the form of microcapsules or implants according to claim 3 or 4, **characterized in that** the PLGA is prepared from 70 to 80 % lactide and 20 to 30 % glycolide.

6. Composition in the form of microcapsules or implants according to one of claims 1 to 5, **characterized in that** the biodegradable polymer is a copolymer of L-lactide and glycolide.

7. Composition in the form of microcapsules or implants comprising at least one biodegradable polymeric or copolymeric excipient or a mixture of such excipients, and at least one water-soluble active substance of high specific surface area.

8. Composition in the form of microcapsules or implants according to claim 7, **characterized in that** the specific surface area of the active substance is greater than 2 m²/g and preferably greater than 3 m²/g.

9. Composition in the form of microcapsules or implants according to claim 8, **characterized in that** the specific surface area of the active substance is greater than 5 m²/g and preferably greater than 10 m²/g.

10. Composition in the form of microcapsules or implants according to claim 9, **characterized in that** the specific surface area of the active substance is greater than 20 m²/g and preferably greater than 30 m²/g.

11. Composition in the form of microcapsules or implants according to any one of claims 7 to 10, **characterized in that** the viscosity of the polymer or copolymer is comprised between 0.5 and 1.6 dl/g in CHCl₃, et preferably comprised between 0.9 and 1.6 dl/g in CHCl₃.

12. Composition in the form of microcapsules or implants according to any one of claims 7 to 11, **characterized in that** the polymer or copolymer presents an hydrophilic character, the acid number of the latter being greater than 1 meq of KOH per gram of polymer or copolymer, and preferably greater than 1.2 or 1.5 meq of KOH per gram of polymer or copolymer.

13. Composition in the form of microcapsules or implants according to any one of claims 7 to 12, **characterized in that** the polymer or copolymer is a PLGA, and preferably a PLGA prepared from 70 to 80 % lactide and 20 to 30 % glycolide.

14. Composition in the form of microcapsules or implants according to any one of claims 1 to 13, **characterized in that** the active substance is a protein or a peptide.

15. Composition in the form of microcapsules or implants according to one of the preceding claims, **characterized in that** the active substance is selected from the group consisting of the following substances: triptorelin or one of its salts, particularly triptorelin acetate, lanreotide or one of its salts, particularly lanreotide acetate, octreotide or one of its salts, particularly octreotide acetate or pamoate, a compound with LH-RH activity, such as triptorelin, goserelin, leuprorelin, buserelin or their salts, an LH-RH antagonist, a GPIIb/IIIa antagonist, a compound with a similar activity to a GPIIb/IIIa antagonist, erythropoietin (EPO) or one of its analogues, the various types of interferon-α, interferon-β or -γ, somatostatin, a somatostatin derivative, a somatostatin analogue, insulin, a growth hormone, a growth hormone releasing factor (GRF), a growth hormone releasing peptide (GHRP), an epidermal growth factor (EGF), a melanocyte stimulating hormone (MSH), a thyrotropin releasing hormone (TRH) or one of its salts or derivatives, a thyroid stimulating hormone (TSH), a luteinizing hormone (LH), a follicle stimulating hormone (FSH), a parathyroid hormone (PTH) or one of its derivatives, a lysozyme hydrochloride, a parathyroid hormone related peptide (PTHrp), an N-terminal peptide fragment (position 1→34) of human PTH hormone, vasopressin or one of its derivatives, oxytocin, calcitonin, a calcitonin derivative with a similar activity to that of calcitonin, a calcitonin gene related peptide (CGRP), glucagon, a peptide similar to glucagon (GLP), gastrin, a gastrin releasing peptide (GRP), secretin, pancreozymin, cholecystokinin, angiotensin, human placental lactogen, human chorionic gonadotropin (HCG), enkephalin, an enkephalin derivative, colony stimulating factor (CSF), endorphin, kyotorphin, interleukins, for example interleukin-2, tuftsin, thymopoietin, thymostimulin, thymic humoral factor (THF), thymic serum factor (TSF), a derivative of thymic serum factor (TSF), thymosin, thymic factor X, tumour necrosis factor (TNF), motilin, bombesin or one of its derivatives, prolactin, neurotensin, dynorphin, caerulein, substance P, urokinase, asparaginase, bradykinin, kallikrein, nerve growth factor, a blood clotting factor, polymixin B, colistin, gramicidin, bacitracin, a protein synthesis stimulating peptide, an endothelin antagonist or one of its salts or derivatives, a vasoactive intestinal polypeptide (VIP), adrenocorticotropic hormone (ACTH) or one of its fragments, a platelet derived growth factor (PDGF), a bone morphogenetic protein (BMP), a pituitary adenylate cyclase activating polypeptide (PACAP), neuropeptide Y (NPY), peptide YY (PYY), a gastric inhibitory polypeptide (GIP) and polynucleotides, especially double-stranded RNAs.

16. Composition in the form of microcapsules or implants according to one of claims 1 to 15, **characterized in that** the active substance is chosen from the group consisting of triptorelin acetate, lanreotide acetate or octreotide acetate.

17. Process for the preparation of composition according to one of the preceding claims comprising the preparation of a water-soluble substance of high specific surface area, comprising the following steps:
- a step of lyophilization of the water-soluble substance comprising the rapid immersion of a dilute solution of said water-soluble substance in a medium the temperature of which is below -50°C, and preferably below -70°C;
- optionally a grinding step, which preferably comprises ultrasonic grinding.

18. Process according to claim 17, **characterized in that** the dilute solution is a solution at a concentration of less than half the saturation concentration.

19. Process according to claim 17 or 18, **characterized in that** the water-soluble substance has a saturation concentration of at least 200 g/l and **in that** the dilute solution is a solution whose concentration is less than a quarter of the saturation concentration.

20. Process according to one of claims 17 to 19, **characterized in that** the rapid immersion is carried out after a step for micronization of the solution of active substance, said micronization step preferably consists in passing the solution through an atomizer.

21. Composition according to claims 1 to 16 comprising an active substance, preferably a protein or a peptide, as obtained by a process according to one of claims 17 to 20.

22. Composition according to claim 21, **characterized in that** the active substance is selected from the group consisting of the following substances: triptorelin or one of its salts, particularly triptorelin acetate, lanreotide or one of its salts, particularly lanreotide acetate, octreotide or one of its salts, particularly octreotide acetate, a compound with LH-RH activity, such as triptorelin, goserelin, leuprorelin, buserelin or their salts, an LH-RH antagonist, a GPIIb/IIIa antagonist, a compound with a similar activity to a GPIIb/IIIa antagonist, erythropoietin (EPO) or one of its analogues, the various types of interferon-α, interferon-β or -γ, somatostatin, a somatostatin derivative, a somatostatin analogue, insulin, a growth hormone, a growth hormone releasing factor (GRF), a growth hormone releasing peptide (GHRP), an epidermal growth factor (EGF), a melanocyte stimulating hormone (MSH), a thyrotropin releasing hormone (TRH) or one of its salts or derivatives, a thyroid stimulating hormone (TSH), a luteinizing hormone (LH), a follicle stimulating hormone (FSH), a parathyroid hormone (PTH) or one of its derivatives, a parathyroid hormone related peptide (PTHrp), an N-terminal peptide fragment (position 1->34) of human PTH hormone, vasopressin or one of its derivatives, oxytocin, calcitonin, a calcitonin derivative with a similar activity to that of calcitonin, a calcitonin gene related peptide (CGRP), glucagon, a peptide similar to glucagon (GLP), gastrin, a gastrin releasing peptide (GRP), secretin, pancreozymin, cholecystokinin, angiotensin, human placental lactogen, human chorionic gonadotropin (HCG), enkephalin, an enkephalin derivative, colony stimulating factor (CSF), endorphin, kyotorphin, interleukins, for example interleukin-2, tuftsin, thymopoietin, thymostimulin, thymic humoral factor (THF), thymic serum factor (TSF), a derivative of thymic serum factor (TSF), thymosin, thymic factor X, tumour necrosis factor (TNF), motilin, bombesin or one of its derivatives, prolactin, neurotensin, dynorphin, caerulein, substance P, urokinase, asparaginase, bradykinin, kallikrein, nerve growth factor, a blood clotting factor, polymixin B, colistin, gramicidin, bacitracin, a protein synthesis stimulating peptide, an endothelin antagonist or one of its salts or derivatives, a vasoactive intestinal polypeptide (VIP), adrenocorticotropic hormone (ACTH) or one of its fragments, a platelet derived growth factor (PDGF), a bone morphogenetic protein (BMP), a pituitary adenylate cyclase activating polypeptide (PACAP), neuropeptide Y (NPY), peptide YY (PYY), a gastric inhibitory polypeptide (GIP) and polynucleotides, especially double-stranded RNAs.

23. Composition according to claim 21 comprising triptorelin acetate, lanreotide acetate or octreotide acetate as obtained by a process according to one of the claims as active ingredient.

24. Composition according to claim 21 comprising double-stranded RNA, preferably polyadenylic acid complexed with polyuridylic acid, as obtained by a process according to one of the claims as active ingredient.

## Patentansprüche

1. Zusammensetzung in Form von Mikrokapseln oder Implantaten, umfassend einen Hilfsstoff oder eine Mischung von biologisch abbaubaren polymeren oder copolymeren Hilfsstoffen und einen Wirkstoff oder eine Mischung von Wirkstoffen, wobei die Mikrokapseln oder die Implantate den Wirkstoff oder die Mischung von Wirkstoffen über einen langanhaltenden Zeitraum, der bis zu drei Monaten oder mehr gehen kann, gemäß einem im Wesentlichen einphasigen Profil freisetzen können, wobei diese Zusammensetzung **dadurch gekennzeichnet ist, dass**:
- wenn die Zusammensetzung in Form von Mikrokapseln vorliegt, die Hilfsstoffe Copolymere umfassen, deren Viskosität zwischen 0,9 dl/g und 1,6 dl/g in CHCl₃ beträgt, und das Verfahren zur Herstellung dieser Mikrokapseln keinen Schritt des Schmelzens dieser Mikrokapseln umfasst,
- oder wenn die Zusammensetzung in Form von Implantaten vorliegt, die Viskosität dieser Polymere oder Copolymere zwischen 0,5 dl/g und 1,6 dl/g in CHCl₃ beträgt.

2. Zusammensetzung in Form von Mikrokapseln oder Implantaten nach Anspruch 1, **dadurch gekennzeichnet, dass** der biologisch abbaubare polymere oder copolymere Hilfsstoff eine inhärente Viskosität von mindestens gleich 0,9 dl/g in CHCl₃ besitzt.

3. Zusammensetzung in Form von Mikrokapseln oder Implantaten nach einem der Ansprüche 1 oder 2, **dadurch gekennzeichnet, dass** das biologisch abbaubare Polymer ein Copolymer von Lactid und Glycolid (PLGA) ist.

4. Zusammensetzung nach Anspruch 3, **dadurch gekennzeichnet, dass** die Säurezahl des Polymers oder Copolymers höher als 1 meq KOH pro Gramm Polymer oder Copolymer und vorzugsweise höher als 1,2 oder 1,5 meq KOH pro Gramm Polymer oder Copolymer ist.

5. Zusammensetzung in Form von Mikrokapseln oder Implantaten nach Anspruch 3 oder 4, **dadurch gekennzeichnet, dass** das PLGA ausgehend von 70 bis 80 % Lactid und 20 bis 30 % Glycolid hergestellt ist.

6. Zusammensetzung in Form von Mikrokapseln oder Implantaten nach einem der Ansprüche 1 bis 5, **dadurch gekennzeichnet, dass** das biologisch abbaubare Polymer ein Copolymer von L-Lactid und Glycolid ist.

7. Zusammensetzung in Form von Mikrokapseln oder Implantaten, umfassend mindestens einen Hilfsstoff oder eine Mischung von biologisch abbaubaren polymeren oder copolymeren Hilfsstoffen und mindestens einen wasserlöslichen Wirkstoff mit einer hohen spezifischen Oberfläche.

8. Zusammensetzung in Form von Mikrokapseln oder Implantaten nach Anspruch 7, **dadurch gekennzeichnet, dass** die spezifische Oberfläche des Wirkstoffs größer als 2 m²/g und vorzugsweise größer als 3 m²/g ist.

9. Zusammensetzung in Form von Mikrokapseln oder Implantaten nach Anspruch 8, **dadurch gekennzeichnet, dass** die spezifische Oberfläche des Wirkstoffs größer als 5 m²/g und vorzugsweise größer als 10 m²/g ist.

10. Zusammensetzung in Form von Mikrokapseln oder Implantaten nach Anspruch 9, **dadurch gekennzeichnet, dass** die spezifische Oberfläche des Wirkstoffs größer als 20 m²/g und vorzugsweise größer als 30 m²/g ist.

11. Zusammensetzung in Form von Mikrokapseln oder Implantaten nach einem der Ansprüche 7 bis 10, **dadurch gekennzeichnet, dass** die Viskosität des Polymers oder des Copolymers zwischen 0,5 und 1,6 dl/g in CHCl₃ und vorzugsweise zwischen 0,9 und 1,6 dl/g in CHCl₃ beträgt.

12. Zusammensetzung in Form von Mikrokapseln oder Implantaten nach einem der Ansprüche 7 bis 11, **dadurch gekennzeichnet, dass** das Polymer oder das Copolymer einen hydrophilen Charakter besitzt, wobei die Säurezahl des letzteren höher als 1 meq KOH pro Gramm Polymer oder Copolymer und vorzugsweise höher als 1,2 oder 1,5 meq KOH pro Gramm Polymer oder Copolymer ist.

13. Zusammensetzung in Form von Mikrokapseln oder Implantaten nach einem der Ansprüche 7 bis 12, **dadurch gekennzeichnet, dass** das Polymer oder Copolymer ein PLGA, vorzugsweise ein PLGA, das ausgehend von 70 bis 80 % Lactid und 20 bis 30 % Glycolid hergestellt ist, ist.

14. Zusammensetzung in Form von Mikrokapseln oder Implantaten nach einem der Ansprüche 1 bis 13, **dadurch gekennzeichnet, dass** der Wirkstoff ein Protein oder ein Peptid ist.

15. Zusammensetzung in Form von Mikrokapseln oder Implantaten nach einem der vorhergehenden Ansprüche, **dadurch gekennzeichnet, dass** der Wirkstoff aus der Gruppe ausgewählt ist, die aus den folgenden Substanzen besteht: Triptorelin oder eines seiner Salze, insbesondere Triptorelinacetat, Lanreotid oder eines seiner Salze, insbesondere Lanreotidacetat, Octreotid oder eines seiner Salze, insbesondere Octreotidacetat oder
- pamoat, eine Verbindung mit einer LH-RH-Aktivität, wie Triptorelin, Goserelin, Leuprorelin, Buserelin oder deren Salze, ein LH-RH-Antagonist, ein GPIIb/IIIa-Antagonist, eine Verbindung mit einer ähnlichen Aktivität wie ein GPIIb/IIIa-Antagonist, Erythropoietin (EPO) oder eines seiner Analoga, die verschiedenen α-Interferone, β- oder y-Interferon, Somatostatin, ein Somatostatinderivat, ein Somatostatinanalogon, Insulin, ein Wachstumshormon, ein Wachstumshormon freisetzender Faktor (GRF), ein Wachstumshormon freisetzendes Peptid (GHRP), ein Epidermalwachstumsfaktor (EGF), ein Melanocyten-stimulierendes Hormon (MSH), ein Thyrotropin freisetzendes Hormon (TRH) oder eines seiner Salze oder Derivate, ein Thyroidea-stimulierendes Hormon (TSH), ein luteinisierendes Hormon (LH), ein die Follikel stimulierendes Hormon (FSH), ein Parathyroidhormon (PTH) oder eines seiner Derivate, ein Lysozymhydrochlorid, ein Parathyroidhormon-related-Peptid (PTHrp), ein N-terminales Peptidfragment (Position 1 → 34) des menschlichen PTH-Hormons, Vasopressin oder eines seiner Derivate, Oxytocin, Calcitonin, ein Derivat von Calcitonin mit einer ähnlichen Wirkung wie Calcitonin, ein Calcitoningen-related-Peptid (CGRP), Glucagon, ein dem Glucagon ähnliches Peptid (GLP), Gastrin, ein Gastrin freisetzendes Peptid (GRP), Secretin, Pancreozymin, Cholecystokinin, Angiotensin, Lactogen der menschlichen Plazenta, das humane Chorion-Gonadotropin (HCG), Enkephalin, ein Derivat von Enkephalin, der Kolonie-stimulierende Faktor (CSF), Endorphin, Kyotorphin, Interleukine, beispielsweise Interleukin 2, Tuftsin, Thymopoietin, Thymosthymlin, der Thymus-Humoral-Faktor (THF), der Thymus-Serum-Faktor (FTS), ein Derivat des Thymus-Serum-Faktors (FTS), Thymosin, der Thymusfaktor X, der Tumornekrosefaktor (TNF), Motilin, Bombesin oder eines seiner Derivate, Prolactin, Neurotensin, Dynorphin, Caerulein, Substanz P, Urokinase, Asparaginase, Bradykinin, Kallikrein, der Nervenwachstumsfaktor, ein Blutgerinnungsfaktor, Polymixin B, Colistin, Gramicidin, Bacitracin, ein die Proteinsynthese stimulierendes Peptid, ein Endothelin-Antagonist oder eines seiner Salze oder Derivate, ein vasoaktives Intestinalpolypeptid (VIP), adrenocorticotropes Hormon (ACTH) oder eines seiner Fragmente, ein von den Blutplättchen abgeleiteter Wachstumsfaktor (PDGF), ein morphogenetisches Knochenprotein (BMP), ein pituitäres Adenylatcyclase-aktivierendes Polypeptid (PACAP), das Neuropeptid Y (NPY), das Peptid YY (PYY), ein gastroinhibitorisches Polypeptid (GIP) und Polynucleotide, insbesondere zweisträngige RNA.

16. Zusammensetzung in Form von Mikrokapseln oder Implantaten nach einem der Ansprüche 1 bis 15, **dadurch gekennzeichnet, dass** die aktive Substanz aus einer Gruppe ausgewählt ist, die aus Triptorelinacetat, Lanreotidacetat oder Octreotidacetat besteht.

17. Verfahren zur Herstellung einer Zusammensetzung nach einem der vorhergehenden Ansprüche, umfassend die Herstellung eines wasserlöslichen Stoffs, der eine hohe spezifische Oberfläche aufweist, umfassend die folgenden Schritte:
- einen Schritt der Lyophilisierung des wasserlöslichen Stoffs, umfassend eine schnelle Härtung einer verdünnten Lösung dieses wasserlöslichen Stoffs in einem Medium mit einer Temperatur unter -50°C und vorzugsweise unter -70°C;
- gegebenenfalls einen Zerkleinerungsschritt, der vorzugsweise eine Ultraschallzerkleinerung umfasst.

18. Verfahren nach Anspruch 17, **dadurch gekennzeichnet, dass** die verdünnte Lösung eine Lösung mit einer Konzentration ist, die kleiner als die Hälfte der Sättigungskonzentration ist.

19. Verfahren nach Anspruch 17 oder 18, **dadurch gekennzeichnet, dass** der wasserlösliche Stoff eine Sättigungskonzentration von mindestens 200 g/l aufweist und dass die verdünnte Lösung eine Lösung mit einer Konzentration ist, die kleiner als ein Viertel der Sättigungskonzentration ist.

20. Verfahren nach einem der Ansprüche 17 bis 19, **dadurch gekennzeichnet, dass** die schnelle Härtung nach einem Schritt der Mikronisierung der Wirkstofflösung vorgenommen wird, wobei dieser Mikronisierungsschritt vorzugsweise in dem Durchlauf der Wirkstofflösung durch einen Zerstäuber besteht.

21. Zusammensetzung nach Anspruch 1 bis 16, umfassend einen Wirkstoff, vorzugsweise ein Protein oder ein Peptid, wie er mit Hilfe eines Verfahrens nach einem der Ansprüche 17 bis 20 erhalten wird.

22. Zusammensetzung nach Anspruch 21, **dadurch gekennzeichnet, dass** der Wirkstoff aus der Gruppe ausgewählt ist, die aus den folgenden Substanzen besteht: Triptorelin oder eines seiner Salze, insbesondere Triptorelinacetat, Lanreotid oder eines seiner Salze, insbesondere Lanreotidacetat, Octreotid oder eines seiner Salze, insbesondere Octreotidacetat, eine Verbindung mit einer LH-RH-Aktivität, wie Triptorelin, Goserelin, Leuprorelin, Buserelin oder deren Salze, ein LH-RH-Antagonist, ein GPIIb/IIIa-Antagonist, eine Verbindung mit einer ähnlichen Aktivität wie ein GPIIb/IIIa-Antagonist, Erythropoietin (EPO) oder eines seiner Analoga, die verschiedenen α-Interferone, β- oder γ-Interferon, Somatostatin, ein Somatostatinderivat, ein Somatostatinanalogon, Insulin, ein Wachstumshormon, ein Wachstumshormon freisetzender Faktor (GRF), ein Wachstumshormon freisetzendes Peptid (GHRP), ein Epidermalwachstumsfaktor (EGF), ein Melanocyten-stimulierendes Hormon (MSH), ein Thyrotropin freisetzendes Hormon (TRH) oder eines seiner Salze oder Derivate, ein Thyroidea-stimulierendes Hormon (TSH), ein luteinisierendes Hormon (LH), ein die Follikel stimulierendes Hormon (FSH), ein Parathyroidhormon (PTH) oder eines seiner Derivate, ein Parathyroidhormon-related-Peptid (PTHrp), ein N-terminales Peptidfragment (Position 1 → 34) des menschlichen PTH-Hormons, Vasopressin oder eines seiner Derivate, Oxytocin, Calcitonin, ein Derivat von Calcitonin mit einer ähnlichen Wirkung wie Calcitonin, ein Calcitoningen-related-Peptid (CGRP), Glucagon, ein dem Glucagon ähnliches Peptid (GLP), Gastrin, ein Gastrin freisetzendes Peptid (GRP), Secretin, Pancreozymin, Cholecystokinin, Angiotensin, Lactogen der menschlichen Plazenta, das humane Chorion-Gonadotropin (HCG), Enkephalin, ein Derivat von Enkephalin, der Kolonie-stimulierende Faktor (CSF), Endorphin, Kyotorphin, Interleukine, beispielsweise Interleukin 2, Tuftsin, Thymopoietin, Thymosthymlin, der Thymus-Humoral-Faktor (THF), der Thymus-Serum-Faktor (FTS), ein Derivat des Thymus-Serum-Faktors (FTS), Thymosin, der Thymusfaktor X, der Tumornekrosefaktor (TNF), Motilin, Bombesin oder eines seiner Derivate, Prolactin, Neurotensin, Dynorphin, Caerulein, Substanz P, Urokinase, Asparaginase, Bradykinin, Kallikrein, der Nervenwachstumsfaktor, ein Blutgerinnungsfaktor, Polymixin B, Colistin, Gramicidin, Bacitracin, ein die Proteinsynthese stimulierendes Peptid, ein Endothelin-Antagonist oder eines seiner Salze oder Derivate, ein vasoaktives Intestinalpolypeptid (VIP), adrenocorticotropes Hormon (ACTH) oder eines seiner Fragmente, ein von den Blutplättchen abgeleiteter Wachstumsfaktor (PDGF), ein morphogenetisches Knochenprotein (BMP), ein pituitäres Adenylatcyclase-aktivierendes Polypeptid (PACAP), das Neuropeptid Y (NPY), das Peptid YY (PYY), ein gastroinhibitorisches Polypeptid (GIP) und Polynucleotide, insbesondere zweisträngige RNA.

23. Zusammensetzung nach Anspruch 21, umfassend Triptorelinacetat, Lanreotidacetat oder Octreotidacetat, wie mit Hilfe eines Verfahrens nach einem der Ansprüche erhalten, als Wirkstoff.

24. Zusammensetzung nach Anspruch 21, umfassend doppelsträngige RNA, vorzugsweise einen Polyadenylsäurekomplex mit Polyuridylsäure, wie mit Hilfe eines Verfahrens nach einem der Ansprüche erhalten, als Wirkstoff.
